# EUROPEAN PATENT SPECIFICATION

(11) **EP 0 282 143 B1**
(45) Date of publication and mention of the grant of the patent: **15.09.1993**
(21) Application number: 88300026.7
(22) Date of filing: 05.01.1988
(51) Int. Cl.: A61M 25/00

(54) **Catheter and guidewire exchange system**
Katheter und System zum Auswechseln eines Führungsdrahtes
Système d'échange de cathéter et fil de guidage

(30) Priority: 27.02.1987 US 19644
(43) Date of publication of application: 14.09.1988
(73) Proprietor: C.R. BARD, INC., Murray Hill New Jersey 07974 (US)
(72) Inventor: Crittenden, James Frederick, Hollis New Hampshire 03049 (US); Barbere, Michael David, Dunstable Massachusetts 01827 (US); White, Bryan John, Lowell Massachusetts 01852 (US)
(74) Representative: Woodward, John Calvin

(56) References cited:
- GB-A- 2 127 294
- US-A- 3 853 130
- US-A- 4 175 564
- US-A- 4 573 470

## Description

### FIELD OF THE INVENTION

This invention relates to catheters placed in the body of a patient such as in the cardiovascular system and, in particular, to a system for facilitating exchange of such catheters and for transporting such catheters to selected sites within the patient.

### BACKGROUND OF THE INVENTION

Catheters are placed at various locations within a patient for a wide variety of purposes and medical procedures. For example only, one type of catheter is a balloon dilatation catheter which is used in the treatment of a vascular stenosis. Such a catheter has a balloon at its distal end which is intended to be placed, in a deflated condition, within the stenosis, and then inflated while in the stenosis to expand radially the stenosed lumen of the blood vessel. Typically, the placement of such catheters involves the use of a guidewire which may be advanced through the patient's vasculature to the location which is to be treated. The catheter, which has a lumen adapted to receive the guidewire, then is advanced over the wire, or the wire and the catheter may be advanced in unison with the wire protruding from the distal end of the catheter. In either case, the wire serves to guide the catheter to the location to be treated.

It often becomes necessary, in the performance of a catheter procedure, to exchange the indwelling catheter for another catheter, for example, for a catheter having a different size balloon. In a typical catheter exchange, the guidewire first is removed from the lumen of the indwelling catheter Then a longer exchange wire, usually about twice the length of the catheter, is passed through the catheter to replace the original wire. Then, while holding the exchange wire by its proximal end to maintain it in place, the catheter is withdrawn proximally from the blood vessel over the exchange wire. After the first catheter has been removed, the next catheter then is threaded onto the proximal end of the exchange wire and is advanced along the exchange wire and through the patient's blood vessels until the distal end of the catheter is located as desired. The exchange wire may be permitted to remain in place or may be exchanged for a shorter, conventional length guidewire.

A device, referred to as the "monorail" system, has been proposed recently which would modify the foregoing catheter exchange technique. In the proposed monorail system, the catheter is formed so that the guidewire is located outside of the catheter except for a short segment at the distal end of the catheter, which passes over the wire. The distal segment of the catheter has a short lumen which extends from the distal tip of the catheter to a more proximally located opening near the distal tip. In use, the guidewire is placed initially in the patient's vascular system. The distal segment of the catheter then is threaded onto the wire. The catheter can be advanced alongside the wire with its distal segment being attached to and guided along the wire. The catheter can be removed and exchanged for another catheter without the use of the usual double length exchange wire and without requiring withdrawal of the initially placed guidewire.

Although the proposed monorail catheter system may avoid the requirement for using a long exchange wire, it presents several difficulties. For example, it is not possible to exchange guidewires in an indwelling catheter, should that be desired. Additionally, the device presents a potential for damaging the delicate inner surface of an artery from a tension load applied to the guidewire which would tend to straighten the artery. Also, there is an increased risk of guidewire entangelement in those procedures where multiple guidewires are used, because the guidewires are exposed within the blood vessel. It is among the general objects of the invention to provide an improved device which overcomes the foregoing difficulties.

US-A-3.853.130 (Sheridan) discloses a catheter with a fitting in the form of a vacuum controller attached to its proximal end. It also discloses the use of a protective sheath split along its whole length to enable the sheath to be peeled from the catheter after insertion in the patient. This document has been used for the delineation of Claims 1 and 19.

US-A-4.175.564 (Kwak) also discloses an introducer split along its whole length which is used to insert a nasal gastric tube into a patient's esophagus and can be peeled from said tube when it has reached its desired location in the patient.

In both of these prior patents, the purpose of the split tube is to act as an introducer for a catheter or a tube, the tube being split along its whole length to allow it to be subsequently peeled off the tube.

The prior art therefore discloses examples of catheters comprising an elongate catheter shaft having proximal and distal ends with a lumen extending longitudinally therethrough and a connector fitting mounted to the proximal end of the catheter.

The present invention is characterised over the prior art in that the catheter is adapted for accommodating only a guidewire, the system being adapted to enable catheter exchanges without using exchange wire or guidewire exchanges and in that the catheter shaft further includes a guidewire lumen extending longitudinally therethrough and a single, linear longitudinally extending slit formed in the catheter shaft and extending from a location in proximity to said fitting along the catheter shaft and in communication with the guidewire lumen to enable transverse access to the guidewire lumen through the catheter shaft, the slit extending along the major portion of the length of the catheter shaft.

A catheter system is provided with a guidewire lumen formed through the elongate body of the catheter. The guidewire lumen is constructed to receive and retain the guidewire fully within and along the indwelling length of the catheter but enables the guidewire to extend out of the guidewire lumen for most of the proximal portion of the catheter which is exposed externally of the patient. The guidewire lumen extends along the length of the catheter, from its proximal end to its distal end. The catheter body is slit longitudinally along the guidewire lumen. The slit enables the guidewire to extend transversely into or out of the lumen through the slit, and at any location along the length of the slit. The slit may extend the full length of the catheter, to the distal tip of the catheter except in those catheters which have an encircling member at the distal tip, such as a balloon, in which case the slit terminates proximally of the encircling member, thus leaving a short unslit distal segment. The system of the present invention includes a guiding device which is slidably carried by the catheter and which serves to merge or to separate the guidewire and catheter lumen at any location along the length of the guidewire or catheter. The guide device has two proximal inlet passageways which merge into a common distal outlet. The inlets receive the separate guidewire and catheter which exit from the common outlet in a merged form in which the guidewire is disposed within the lumen of the catheter. The relative direction of movement of the catheter, guidewire and guide device determine whether the guiding device merges or separates the catheter and the wire.

When using the system, the guidewire first is placed in the patient's vascular system with the distal end of the guidewire placed at the desired location in the patient's blood vessel. The catheter is threaded onto the guidewire which passes through the guidewire lumen until the proximal end of the guidewire enters the guide device where it is guided transversely through the slit in the catheter and out of the guidewire lumen of the catheter. The guide device is constructed to engage and spread the slit in the catheter body while guiding the guidewire out of the guidewire lumen. The catheter may be advanced into the patient by securing the position of the guidewire relative to the guiding device and advancing the catheter through the guiding device which spreads the slit and guides the catheter so that the guidewire lumen wraps about and embraces the guidewire. The catheter and guidewire exit from the common branch of the guide device with the guidewire in the lumen. In reverse operation, when the catheter is withdrawn, the guide device separates the catheter and guidewire to enable either the guidewire or the catheter to be withdrawn independently through its respective branch in the guide device. In another mode of operation, the guide device may be operated as a zipper by holding the catheter and guidewire stationary and by advancing the guide device either in a distal direction to separate the wire and catheter or in a proximal direction to merge them together. The wire thus is enclosed within the catheter lumen along the full length of the indwelling portion of the catheter. Thus, it is possible to remove the guidewire and replace it with another wire if desired without removing the catheter from the patient. Except for the distal end of the guidewire, which protrudes out of the distal tip of the catheter, the guidewire is covered at all times while within the blood vessel, thus reducing the risk of injury to the blood vessel intima as well as the risk of wire entanglement in multiple wire procedures.

It is among the objects of the invention to provide an improved catheter and guidewire system.

Another object of the invention is to provide a guidewire and catheter system in which the portion of the guidewire which extends long the indwelling portion of the catheter is contained within the catheter but in which the externally disposed portion of the catheter does not contain the guidewire.

A further object of the invention is to provide a system of the type described which enables guidewire exchanges to be made through the indwelling catheter. A further object of the invention is to provide a system of the type described which enables catheter exchanges to be made without using exchange wires.

Another object of the invention is to provide a system of the type described in which the guidewire resides in the indwelling portion of the catheter and separates from the catheter externally of the patient to provide a grippable portion for the physician.

A further object of the invention is to provide a system of the type described in which the guidewire exits transversely of the catheter through a slit formed in the catheter wall.

### BRIEF DESCRIPTION OF THE DRAWINGS

The foregoing and other objects and advantages of the invention will be appreciated more fully from the following further description thereof with reference to the accompanying drawings.
FIG. 1 is an illustration of one embodiment of the catheter, guidewire and guide member of the invention in an assembled configuration;
FIG. 2 is a sectional illustration of the catheter and guidewire as seen along the line 2-2 of FIG. 1;
FIG. 3 is a sectional illustration of the catheter, guidewire and guide member as seen along the line 3-3 of FIG. 1;
FIG. 4 is a sectional illustration of the catheter, guide member and guidewire as seen along the line 4-4 of FIG. 1;
FIG. 5 is a sectional illustration of the catheter and guidewire as seen along the line 5-5 of FIG. 1;
FIG. 6 is a sectional illustration of the catheter and guidewire as seen along the line 6-6 of FIG. 1;
FIG. 7 is an enlarged sectional illustration of the guide member as seen along the line 7-7 of FIG. 8;
FIG. 8 is an end view of the distal end of the guide member;
FIG. 9 is an illustration of the guide member with the guidewire and catheter extending therethrough and illustrating the manner in which the guide member merges the guidewire and catheter;
FIG. 10 is an illustration of the juncture region of the guide member with the guidewire and catheter in place as seen along the line 10-10 of FIG. 9;
FIG. 11 is a broken and partially enlarged illustration of an embodiment of the invention in which the slit in the guidewire lumen extends fully to the distal tip of the catheter; and
FIG. 12 is a broken and partially enlarged illustration of a two lumen catheter in which the slit in the guidewire lumen extends to the distal tip and in which at least one additional, unslit lumen is provided.

### DESCRIPTION OF THE PREFERRED EMBODIMENT

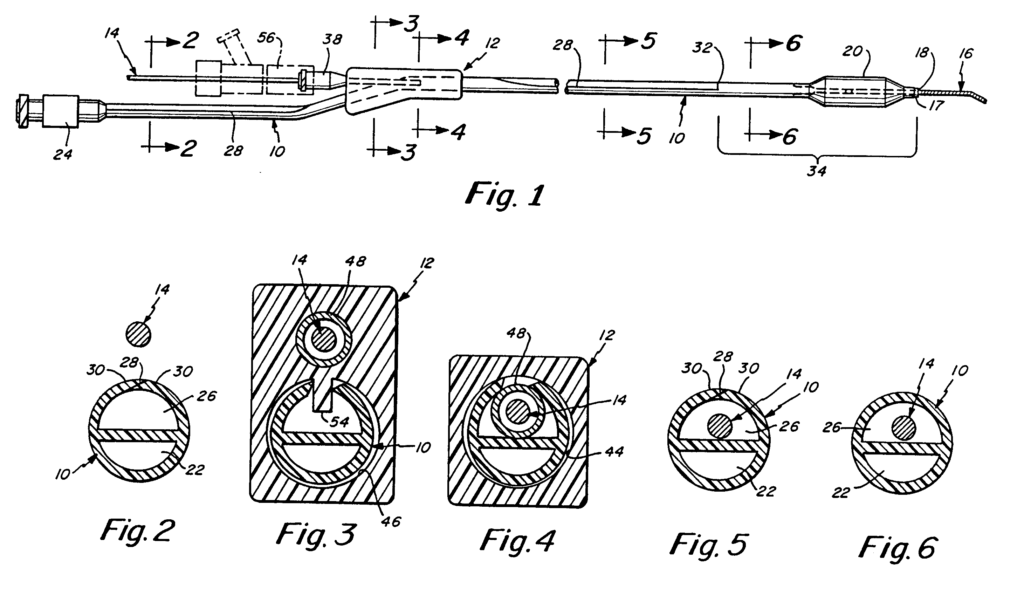

As shown in FIG. 1, the invention includes a catheter, indicated generally by the reference character 10 on which a guide member 12 is slidably mounted. A guidewire 14 is illustrated as extending through the guide member 12. The guide member 12 serves as a juncture in which the catheter 10 and guidewire 14 may be merged or separated so that the portion of the guidewire which extends proximally of the guide member 12 (to the left as seen in FIG. 1) is separated from the catheter 10 and the portion of the guidewire 14 which is located distally of the guide member 12 (to the right as seen in FIG. 1) is contained and housed within the catheter 10 except for the most distal end 16 of the guidewire 14 which may protrude distally out of a distal outlet opening 18 of the catheter 10.

The catheter 10 includes an elongate, flexible, cylindrical main body which may be formed from an extruded plastic material such as, for example, polyethylene. In the embodiment shown in FIG. 1, the catheter 10 is a balloon dilatation catheter used in angioplasty procedures and has a balloon 20 formed near the distal end of the catheter 10 and which encircles the catheter body. The balloon 20 may be inflated and deflated through an inflation lumen 22 formed through the body of the catheter 10. The inflation lumen extends from the proximal end of the catheter, where it communicates with a fitting 24 and extends the length of the catheter, terminating in communication with the interior of the balloon 20. The fitting 24 is intended to be connected to a suitable source of pressurized fluid or suction to inflate or deflate the balloon 20. The catheter 10 includes a second lumen, indicated at 26 which is intended to receive the guidewire 14. The guidewire lumen 26 may extend the full length of the catheter, terminating at the distal outlet 18. The guidewire lumen 26 need not extend completely to the proximal fitting 24, although it may so extend, if desired.

In accordance with the invention, the body of the catheter 10 is formed with a longitudinal slit 28 which, when the catheter 10 is viewed in cross-section (as FIG. 2), may be considered as defining a pair of flaps 30 which normally close together at the slit 28 to define the enclosed guidewire lumen 26. As seen in cross-section in FIG. 2, the slit 28 preferably is formed so that it extends in a generally tangential direction with respect to the generally circular wall of the catheter. The tangentially oriented slit has been found to provide a somewhat better seal and enables fluids at relatively low pressures to flow through the guidewire lumen. This enables the guidewire lumen also to serve as a low pressure infusion lumen, such as for the infusion of drugs under low pressure and relatively low flow rates. The guidewire lumen 26 may be circular in cross-section or may be non-circular; in either case, the cross-sectional dimensions of the guidewire lumen 26 are greater than the cross-sectional dimension of the guidewire 14 to permit relative longitudinal movement between the guidewire 14 and catheter 10.

The proximal end of the slit 28 may terminate at or near the fitting 24. In the embodiment shown in FIG. 1, in which the catheter has an encircling member, such as the balloon 20, at its distal end, the distal end 32 of the slit 28 terminates short of the distal tip 17 of the catheter, thereby leaving a distal segment 34 of the catheter 10 which is unslit and in which the guidewire lumen 26 is defined by a continuous, unslit surrounding wall as shown in FIG. 6. It should be understood, however, that the principals of the invention also are usable with catheters which do not have encircling members at their distal ends and, in those catheters, the slit 28 may extend fully to the distal tip of the catheter, as desired.

The guide member 12 may be molded from a suitably rigid plastic material such as a polycarbonate (e.g., Lexan). The member 12 may be considered as having a proximal end 34 and a distal end 36. An inlet fitting 38 is formed at the proximal end 34 and may be formed with a tapered passage 40 which, in turn, leads into a distally extending bore 42. The bore 42 extends toward a larger diameter common passageway 44 which exits at the distal end 36 of the member 12. The member 12 also includes a catheter passageway 46 which extends at an oblique angle to the bore 42 and passageway 44 and merges with bore 42 and passageway 44 in the region of their juncture. In the illustrative embodiment, a tubular member 48, which may be formed from hypodermic tubing, is mounted securely within the bore 42 and has a distal end 50 which projects into the common passageway 44, adjacent the juncture with the catheter passageway 46. The tube 48 defines a guidewire passageway and communicates the luer fitting 38 with the common passageway 44. A spreader member 54 is formed in the body of the guide member 12 and projects into the catheter passageway 46, the spreader being disposed adjacent the distal end of the catheter passageway.

The guide member 12 may be formed conveniently in two molded halves, separated along the passageways with the tubular member 48 being placed in the passageways, as shown, when the halves of the device 12 are assembled and adhesively bonded together. When the catheter is of the type having an unslit distal segment, it may be placed in its respective channel during assembly of the guide member 12.

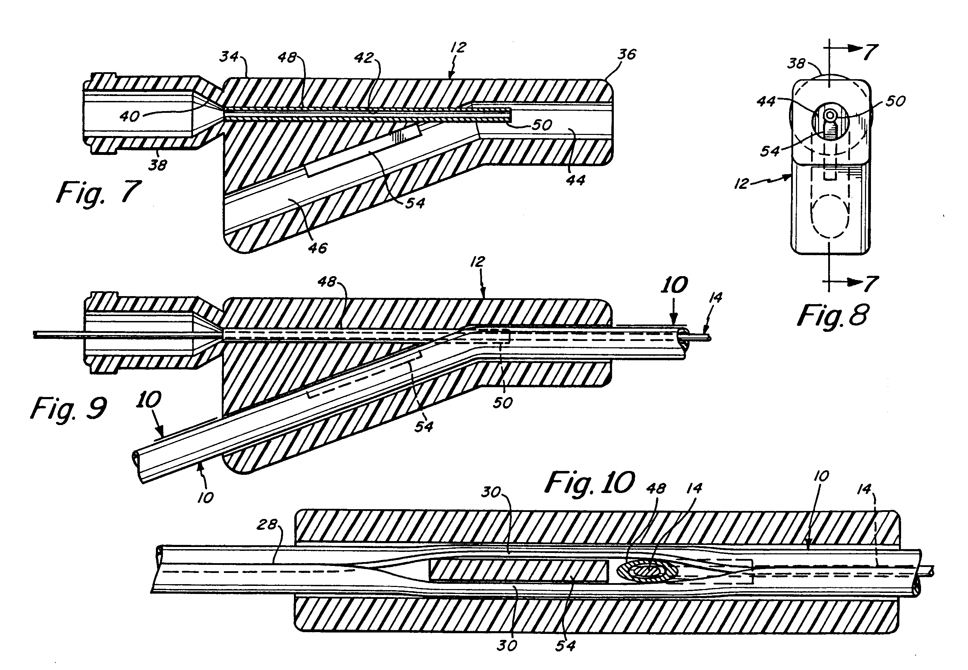

When the catheter 10 and guidewire 14 both pass through the guide member 12, they merge at the juncture of the passageways as indicated in FIGS. 9 and 10. The guidewire 14 extends through the guidewire passageway defined by the tube 48 and into and through the common passageway 44. The catheter 10 extends through the catheter passageway 46 and engages the spreader 54 which extends through the slit 28 in the catheter 10 to spread the flaps 30 apart as indicated in FIG. 10. As the catheter advances distally relative to the guide member 12, it advances to the juncture of the passageways and into the common passageway 44 where the guidewire lumen wraps about the distal end 50 of the tube 48, and the guidewire 14 contained within the distal end 50 as shown in FIG. 3. Thus, when the catheter 10 advances relative to the guide member 12 past the distal end 50 of the tube 48, the guidewire 14 becomes enclosed within the guidewire lumen 26 and the flaps 30 draw together under the influence of the inherent resiliency of the catheter body to close the slit 28 of the catheter. The guidewire is contained within the guidewire lumen from that point to the distal end 17 of the catheter where it may exit through the distal outlet opening 18.

From the foregoing, it will be appreciated that the catheter may be advanced distally relative to the guidewire 14 and guide member 12 so as to cause the catheter to envelop and contain the guidewire 14 completely within the guidewire lumen 26. The guidewire 14 may be advanced distally or withdrawn proximally with respect to the guide member 12 and catheter.

The system may be used by first percutaneously inserting and advancing the guidewire 14 into the patient's blood vessel or other body vessel to be treated so that the distal end 16 of the guidewire 14 is located in the intended place in the patient's vessel. For example only, the invention may be used in connection with steerable guidewires of the type described in US-A-4,545,390 to Leary. The guidewire lumen of the catheter then may be flushed with saline. The distal end of the catheter then is threaded onto the proximal end of the guidewire which is passed through the guide member 12 so that the proximal member of the guidewire 14 protrudes out of the inlet 38. As illustrated in phantom in FIG. 1, a Tuohy-Borst adapter 56 may be carried on the luer fitting 38 of the guide member. The Tuohy-Borst adapter 56 may be tightened down about the guidewire to lock it securely in place with respect to the guide member 12. The physician then may hold the guide member 12 and guidewire 14 stationary relative to each other (by tightening the Tuohy-Borst adapter 56) while he advances the catheter 10 through the guide member 12. As the catheter passes through the guide member 12, it is caused to wrap about the guidewire as described above so that as the catheter is advanced through the patient's blood vessels, it will enclose the guidewire at all times and fully along the indwelling length of the catheter. At the same time, the proximal end of the guidewire 14 always is exposed so that the physician may manipulate it as desired. When the catheter is advanced to the intended location (which may be monitored fluoroscopically) in the patient's blood vessels, the physician will perform the procedure for which the catheter is designed. In the illustrative embodiment of the invention illustrated in FIG. 1, the procedure would be a dilatation procedure to enlarge a stenosed lumen of the blood vessel.

Should the physician have a need to exchange catheters, he may do so by holding the guide member 12 and the protruding proximal end of the guidewire 14 and withdrawing the indwelling catheter. As the catheter is drawn proximally through the guide member 12, the flaps are caused to separate as they are drawn past the distal end 50 of tube 48 and spreader 54. As the distal end of the catheter approaches the guide member 12, both the guide member and catheter are slipped off the proximal end of the guidewire. Another catheter fitted with a guide member 12 then may be placed on the guidewire and advanced along the guidewire and into the patient's blood vessels as was done with the original catheter. Except for the protruding distal end 16, guidewire 14 is contained completely within the indwelling portion of the catheter while the proximal end of the guidewire remains exposed so that it may be controlled from its proximal end by the physician.

Should it be desired to change guidewires while a catheter is present in the patient's blood vessel, that may be done easily by simply withdrawing the guidewire and replacing it with another. During the withdrawal and the replacement, the guidewires will be enclosed within the guidewire lumen 26 of the catheter 10.

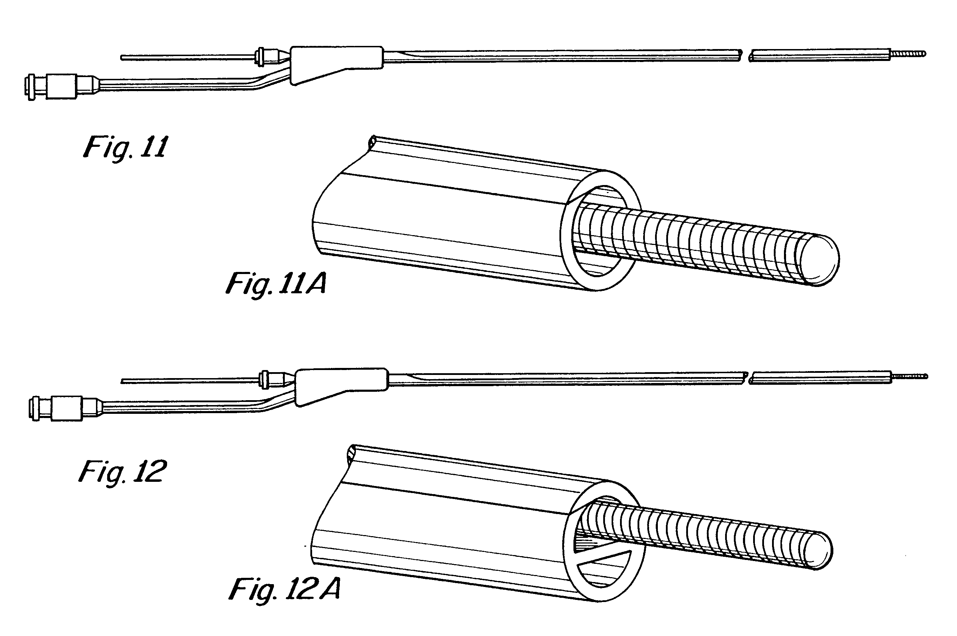

It should be understood that although the invention is described, for purposes of illustration as being used in connection with a balloon dilatation catheter, the invention is not limited to practice with that type of catheter and may be used with any type of catheter, lead or the like which may be placed in a patient by the use of a guidewire. For example, FIG. 11 illustrates a catheter which is free of encircling members at its distal end. In such a catheter, the slit 28 may extend fully to the distal tip of the catheter. Such a catheter need not be prefitted with a guide member because the distal end of the catheter can be inserted directly into the proximal end of the catheter passageway 46 in the guide member 12. For example, the catheter may be a single lumen low pressure infusion catheter (FIG. 11) or a multiple lumen catheter (FIG. 12) in which the guidewire lumen may be used for low pressure fluid communications while a second unslit lumen may be used for higher pressure fluid communications, such as injection of contrast media, pressure monitoring or the like.

Having thus described the invention, what I desire to claim and secure by letters patent is:

## Claims

1. A catheter system comprising an elongate flexible catheter shaft (10) having proximal and distal ends and a lumen (22) extending longitudinally therethrough; a connector fitting (24) mounted to the proximal end of the catheter shaft characterised in that the catheter is adapted for accommodating only a guidewire (14) and in that the system is adapted to enable catheter exchanges without using exchange wires or guidewire exchanges and in that the catheter shaft further includes a guidewire lumen (26) extending longitudinally therethrough and a single, linear longitudinally extending slit (28) formed in the catheter shaft (10) and extending from a location in proximity to said fitting (24) along the catheter shaft (10) and in communication with the guide wire lumen (26) to enable transverse access to the guidewire lumen (26) through the catheter shaft (10), the slit extending along the major portion of the length of the catheter shaft.

2. A catheter system according to claim 1 characterised in that the slit (28) terminates at the distal tip (17) of the catheter.

3. A catheter system according to claim 1 characterized in that the slit (28) has a distal terminal end proximal of the distal end of the catheter thereby defining an unslit distal segment (34) of the catheter.

4. A catheter system according to claim 3 characterized in that an encircling member (20) is mounted to the catheter on the distal segment.

5. A catheter system according to claim 4 characterised in that an encircling member is mounted to the catheter on the distal segment and in that the encircling member comprises a balloon (20), the catheter having a second lumen (22) extending through the shaft for inflation and deflation of the balloon (20).

6. A catheter system according to any preceding claim characterized in that a guidewire (14) extends through the slit (28) into and through the slit lumen, and out the distal end of the catheter.

7. A catheter system according to claim 5 characterised in that the proximal fitting (24) is connected to the proximal end of the catheter and communicates with the second lumen (22).

8. A catheter system according to claim 6 characterised by means (12) for guiding the guidewire (14) transversely into and out of the lumen through the slit (28).

9. A catheter system according to claim 8 characterised in that the guide means comprises a guide member (12) having separate means for receiving the guidewire (14) and the catheter (10) and means for merging the guidewire and the catheter by guiding the guidewire transversely through the slit (28) in the catheter and into the guidewire lumen and for separating the guidewire and catheter by guiding the guidewire out of the lumen transversely through said slit (28).

10. A catheter system according to claim 9 charactersied in that the guidewire (14) is disposed within the lumen distally of the guide means (12) and extends transversely out of the lumen and catheter proximally of the guide means (12).

11. A catheter system according to claim 9 characterised in that the guide means is constructed and arranged to effect said merging and separating in response to relative movement of the guide member (12) to at least one of the guidewire (14) or the catheter (10).

12. A catheter system according to claim 11 characterised in that the guide member (12) comprises a body having a guidewire passageway (48) extending therethrough and a catheter passageway (46) which intersects the guidewire passageway, said catheter passageway and guidewire passageway merging into a common passageway (44).

13. A catheter system according to claim 12 characterised by spreading means (54) adjacent the intersection of said passageways for spreading the slit of the catheter shaft.

14. A catheter system according to claim 13 characterised in that the spreading means comprises a spreader member (54) mounted within the body of the guide member (12) at said intersection and being adapted to project through the slit (28) in the catheter into the lumen to spread the edges of the slit.

15. A catheter system according to claim 14 characterised in that the spreader (54) is located at the distal end of the catheter passageway (46).

16. A catheter system according to claim 6 characterised in that a guide member (12) is mounted on the catheter and has means for receiving a guidewire (14) and means (12) for merging the guidewire and the catheter by guiding the guidewire transversely through the slit (28) in the catheter and into the guidewire lumen and for separating the guidewire and catheter by guiding the guidewire transversely out of the lumen through said slit.

17. A catheter system according to claim 16 characterised in that the guidewire (14) is disposed within the lumen distally of the guide means and extends transversely out of the lumen and catheter proximally of the guide means.

18. A catheter system according to claim 16 characterised in that the guide means is constructed and arranged to effect such merging and separating in response to relative movement of the guide member to at least one of the guidewire or catheter.

19. A catheter system comprising an elongate flexible catheter shaft (10) having proximal and distal ends and a lumen (22) extending longitudinally therethrough; a connector fitting (24) mounted to the proximal end of the catheter shaft characterised in that the catheter is adapted for accommodating only a guidewire (14), in that the system is adapted to enable catheter exchanges without using exchange wires or guidewire exchanges, in that the catheter shaft further includes a guidewire lumen (26) extending longitudinally therethrough and a single, linear longitudinally extending slit (28) formed in the catheter shaft (10) and extending from a location in proximity to said fitting (24) along the catheter shaft (10) and in communication with the guidewire lumen (26) to enable transverse access to the guidewire lumen (26) through the catheter shaft (10), the slit extending along the major portion of the length of the catheter shaft and in that the system further comprises a guide member (12) mounted on the catheter shaft (10) and having a means for receiving a guidewire (14) and means for merging the guidewire and the catheter by guiding the guidewire transversely through the slit (28) in the catheter shaft and into the guidewire lumen and for separating the guidewire and catheter by guiding the guidewire transversely out of the guidewire lumen (26) through said slit; said guide member (12) being constructed and arranged to effect such merging and separating in response to relative movement of the guide member to at least one of the guidewire (14) or catheter (10), said guide member comprising a body having a guidewire passageway (48) extending longitudinally therethrough and a catheter passageway (46) which intersects the guidewire passageway (48), the catheter passageway and guidewire passageway merging into a common passageway (44).

20. A catheter system according to claim 19 characterised by means (54) adjacent the intersection of said passageways for spreading the slit (28) of the catheter shaft.

21. A catheter system according to claim 20 characterised in that the spreading means comprises a spreader member (54) mounted within the body (12) of the guide member at said intersection and being adapted to project through the slit (28) in the catheter into the lumen to spread the edges of the slit.

22. A catheter system according to claim 21 characterised in that the spreader (54) is located at the distal end of the catheter passageway (46).

23. A catheter system according to claim 13 or claim 22 characterised by a tubular member (48) mounted in the guide member adjacent the intersection of the passageways and having a distal end (50) extending toward the common passageway (44), the tubular member (48) being smaller in diameter than the common passageway (44) to enable the guidewire lumen of the catheter to wrap about the tubular member, the tubular member being adapted to receive and guide the guidewire (14) into the the guidewire lumen of the catheter.

## Patentansprüche

1. Kathetersystem mit einem langgestreckten flexiblen Katheterschaft (10), der ein proximales und ein distales Ende und einen diesen sich längs durch ihn hindurch erstreckenden Hohlraum (22) aufweist, wobei am proximalen Ende des Katheterschaftes ein Anschlußstück (24) angebracht ist, dadurch gekennzeichnet, daß der Katheter nur zur Aufnahme eines Führungsdrahtes (14) angepaßt ist, daß das System angepaßt ist, den Austausch von Kathetern ohne Verwendung von Austauschdrähten und ohne Führungsdrahtaustausch zu ermöglichen, und daß der Katheterschaft weiterhin einen sich längs durch ihn hindurch erstreckenden Führungsdraht-Hohlraum (26) sowie einen einzelnen, sich geradlinig längs erstreckenden Schlitz (28) umfaßt, der im Katheterschaft (10) ausgebildet ist und sich von einer Stelle in der Nähe des genannten Anschlußstücks (24) aus entlang dem Katheterschaft (10) und in Verbindung mit dem Führungsdraht-Hohlraum (26) erstreckt, um durch den Katheterschaft (10) hindurch einen queren Zugang zum Führungsdraht-Hohlraum (26) zu ermöglichen, wobei sich der Schlitz entlang dem Hauptabschnitt der Katheterschaftlänge erstreckt.

2. Kathetersystem nach Anpruch 1 dadurch gekennzeichnet, daß der Schlitz (28) am distalen Endstück (17) des Katheters endet.

3. Kathetersystem nach Anspruch 2 dadurch gekennzeichnet, daß der Schlitz (28) ein distales Abschlußende proximal vom distalen Ende des Katheters aufweist, wodurch ein ungeschlitzter distaler Abschnitt (34) des Katheters definiert ist.

4. Kathetersystem nach Anspruch 3 dadurch gekennzeichnet, daß ein ringartiges Glied (20) auf dem distalen Abschnitt des Katheters befestigt ist.

5. Kathetersystem nach Anspruch 4 dadurch gekennzeichnet, daß ein ringartiges Glied auf dem distalen Abschnitt am Katheter befestigt ist und dadurch, daß das ringartige Glied aus einem Ballon (20) besteht, wobei der Katheter einen zweiten Hohlraum (22) aufweist, der sich zum Aufblasen und Entleeren des Ballons (20) durch den Schaft erstreckt.

6. Kathetersystem nach irgendeinem der vorhergehenden Ansprüche, dadurch gekennzeichnet, daß sich ein Führungsdraht (14) durch don Schlitz (28) in den Schlitz-Hohlraum hinein und durch ihn hindurch sowie aus dem distalen Ende des Katheters heraus erstreckt.

7. Kathetersystem nach Anspruch 5 dadurch gekennzeichnet, daß das proximale Anschlußstück (24) mit dem proximalen Ende des Katheters verbunden ist und mit dem zweiten Hohlraum (22) zusammenwirkt.

8. Kathetersystem nach Anspruch 6 durch Mittel (12) gekennzeichnet durch Mittel (12) zur queren Führung des Führungsdrahtes (14) durch den Schlitz (28) in den Hohlraum hinein und aus ihm heraus

9. Kathetersystem nach Anspruch 8, dadurch gekennzeichnet, daß das Führungsmittel ein Führungsglied (12) mit separaten Mitteln zur Aufnahme des Führungsdrahtes (14) und des Katheters (10) und Mittel zum Zusammenführen von Führungsdraht und Katheter aufweist, indem der Führungsdraht quer durch den Schlitz (28) im Katheter und in den Führungsdraht-Hohlraum hinein geführt wird, und zur Trennung von Führungsdrahtes und Katheter durch Führen des Führungsdrahtes quer durch den genannten Schlitz (28) aus dem Hohlraum heraus.

10. Kathetersystem nach Anspruch 9 dadurch gekennzeichnet, daß der Führungsdraht (14) innerhalb des Hohlraumes distal vom Führungsmittel (12) entfernt angeordnet ist und sich quer aus dem Hohlraum und dem Katheter heraus proximal vom Führungsmittel (12) erstreckt.

11. Kathetersystem nach Anspruch 9 dadurch gekennzeichnet, daß das Führungsmittel so ausgelegt und angeordnet ist, um das Zusammenführen und Trennen als Reaktion auf die relative Bewegung des Führungsgliedes (12) zum Führungsdraht (14) oder/oder zum Katheter (10) zu bewirken.

12. Kathetersystem nach Anspruch 11 dadurch gekennzeichnet, daß das Führungsglied (12) einen Körper mit einem sich durch ihn hindurch erstreckenden Führungsdrahtdurchgang (48) sowie einen Katheterdurchgang (46) umfaßt, der den Führungsdrahtdurchgang kreuzt, wobei der genannte Katheter-durchgang und der Führungsdrahtdurchgang in einen gemeinsamen Durchgang (44) einmünden.

13. Kathetersystem nach Anspruch 12, gekennzeichnet durch der Mündung der genannten Durchgänge benachbarte Spreizmittel (54) zum Spreizen des Schlitzes des Katheterschaftes.

14. Kathetersystem nach Anspruch 13 dadurch gekennzeichnet, daß das Spreizmittel ein Spreizglied (54) umfaßt, das im Körper des Führungsgliedes (12) an der genannten Mündung angebracht ist und angepaßt ist, um durch den Schlitz (28) im Katheter in den Hohlraum hineinzuragen, um die Ränder des Schlitzes auseinanderzuspreizen.

15. Kathetersystem nach Anspruch 14 dadurch gekennzeichnet, daß das Spreizglied (54) am distalen Ende des Katheterdurchgangs (46) angeordnet ist.

16. Kathetersystem nach Anspruch 6 dadurch gekennzeichnet, daß das Führungsglied (12) auf dem Katheter befestigt ist und Mittel zur Aufnahme eines Führungsdrahtes (14) aufweist sowie Mittel (12) zum Zusammenführen von Führungsdraht und Katheter durch Führen des Führungsdrahtes quer durch den Schlitz (28) im Katheter und in den Führungsdraht-Hohlraum hinein sowie zur Trennung von Führungsdraht und Katheter durch Führen des Führungsdrahtes quer durch den genannten Schlitz aus dem Hohlraum heraus.

17. Kathetersystem nach Anspruch 16 dadurch gekennzeichnet, daß der Führungsdraht (14) distal vom Führungsmittel im Hohlraum angeordnet ist und sich proximal vom Führungsmittel quer aus dem Hohlraum und dem Katheter heraus erstreckt.

18. Kathetersystem nach Anspruch 16 dadurch gekennzeichnet, daß das Führungsmittel so ausgelegt und angeordnet ist, um ein derartiges Zusammenführen und Trennen als Reaktion auf die relative Bewegung des Führungsgliedes zum Führungsdraht und/oder zum Katheter zu bewirken.

19. Kathetersystem mit einem langgestreckten flexiblen Katheterschaft (10), der ein proximales und ein distales Ende und einen sich längs durch ihn hindurch erstreckenden Hohlraum (22) aufweist, wobei am proximalen Ende des Katheterschaftes ein Anschlußstück (24) befestigt ist, dadurch gekennzeichnet, daß der Katheter nur zur Aufnahme eines Führungsdrahtes (14) angepaßt ist, daß das System angepaßt ist, den Austausch von Kathetern ohne Verwendung von Austauschdrähten und ohne Führungsdrahtaustausch zu ermöglichen, und daß der Katheterschaft weiterhin einen sich längs durch ihn hindurch erstreckenden Führungsdraht-Hohlraum (26) sowie einen einzelnen, sich geradlinig längs erstreckenden Schlitz (28) umfaßt, der im Katheterschaft (10) ausgebildet ist und sich von einer Stelle in der Nähe des genannten Anschlußstücks (24) aus entlang dem Katheterschaft (10) und in Verbindung mit dem Führungsdraht-Hohlraum (26) erstreckt, um durch den Katheterschaft (10) hindurch einen queren Zugang zum Führungsdraht-Hohlraum (26) zu ermöglichen, wobei sich der Schlitz entlang dem Hauptabschnitt der Katheterschaftlänge erstreckt, und daß das System außerdem ein am Katheterschaft befestigtes Führungsglied (12) umfaßt sowie ein Mittel zur Aufnahme eines Führungsdrahtes (14) und Mittel zum Zusammenführen von Führungsdraht und Katheter durch Führen des Führungsdrahtes quer durch den Schlitz (28) im Katheterschaft und in den Führungsdraht-Hohlraum hinein und zum Trennen von Führungsdraht und Katheter durch Führen des Führungsdrahtes quer durch den besagten Schlitz aus dem Führungsdraht-Hohlraum (26) heraus, wobei das genannte Führungsglied (12) so ausgelegt und angeordnet ist, um ein derartiges Zusammenführen und Trennen als Reaktion auf die relative Bewegung des Führungsgliedes zum Führungsdraht (14) und/oder zum Katheter (10) zu bewirken, wobei das besagte Führungsglied einen Körper mit einem sich längs durch hindurch erstreckenden Führungsdrahtdurchgang (48) und einen Katheterdurchgang (46) umfaßt, der den Führungsdrahtdurchgang (48) kreuzt, wobei der Katheterdurchgang und der Führungsdrahtdurchgang in einen gemeinsamen Durchgang (44) einmünden.

20. Kathetersystem nach Anspruch 19, gekennzeichnet durch Mittel (54) nahe der Mündung der genannten Durchgänge zum Auseinanderspreizen des Schlitzes (28) des Katheterschaftes.

21. Kathetersystem nach Anspruch 20 dadurch gekennzeichnet, daß das Spreizmittel ein im Körper (12) des Führungsgliedes (54) an der genannten Mündung befestigtes Spreizglied (54) umfaßt und angepaßt ist, um durch den Schlitz (28) im Katheter hindurch in den Hohlraum hinein zu ragen, um die Ränder des Schlitzes auseinanderzuspreizen.

22. Kathetersystem nach Anspruch 21 dadurch gekennzeichnet, daß das Spreizglied (54) am distalen Ende des Katheterdurchgangs (46) angeordnet ist.

23. Kathetersystem nach Anspruch 13 oder Anspruch 22 durch ein im Führungsglied nahe der Mündung der Durchgänge angebrachtes rohrförmiges Glied (48), welches ein distales Ende (50) aufweist, das sich zum gemeinsamen Durchgang (44) hin erstreckt, wobei das rohrförmige Glied (48) im Durchmesser kleiner ist als der gemeinsame Durchgang (44), damit der Führungsdraht-Hohlraum des Katheters das rohrförmige Glied umhüllen kann, wobei das rohrförmige Glied zur Aufnahme und zur Führung des Führungsdrahtes (14) in den Führungsdraht-Hohlraum des Katheters hinein angepaßt ist.

## Revendications

1. Système de cathéter comprenant un corps de cathéter souple et allongé (10) ayant une extrémité distale et une extrémité proximale, un canal (22) le traversant longitudinalement, et une pièce d'accouplement de connexion (24) monté sur l'extrémité proximale du corps de cathéter,
caractérisé en ce que le cathéter ne convient que pour recevoir un seul fil de guidage (14) et en ce que le système permet l'échange de cathéter sans utiliser de fil d'échange ou d'échange de fil de guidage, et en ce que le corps de cathéter présente de plus un canal pour fil de guidage (26) le traversant longitudinalement et une fente (28) unique linéaire longitudinale pratiquée dans le corps de cathéter (10) depuis un emplacement voisin dudit accouplement (24) tout le long du corps de cathéter (10) et en communication avec le canal pour fil de guidage (26) de manière à permettre l'accès transversal au canal pour fil de guidage (26) à travers le corps du cathéter (10), cette fente s'étendant sur la plus grande partie de la longueur du corps de cathéter.

2. Système de cathéter selon la revendication 1, caractérisé en ce que la fente (28) se termine à l'extrémité distale (17) du cathéter.

3. Système de cathéter selon la revendication 1, caractérisé en ce que l'extrémité distale de la fente (28) est voisine de l'extrémité distale du cathéter, en définissant ainsi un segment distal non fendu (34) du cathéter.

4. Système de cathéter selon la revendication 3, caractérisé en ce qu'un organe de cerclage (20) est monté sur le segment distal du cathéter.

5. Système de cathéter selon la revendication 4, caractérisé en ce que ledit organe de cerclage consiste en un ballon (20), le cathéter ayant un second canal (22) traversant le corps en vue du gonflage et du dégonflage du ballon (20).

6. Système de cathéter selon l'une quelconque des revendications précédentes, caractérisé en ce qu'un fil de guidage (14) traverse la fente (28) en pénétrant dans l'ouverture de la fente en sortant à l'extrémité distale du cathéter.

7. Système de cathéter selon la revendication 5, caractérisé en ce que l'accouplement (24) proximal est connecté à l'extrémité proximale du cathéter et communique avec le second canal (22).

8. Système de cathéter selon la revendication 6, caractérisé par des moyens (12) pour guider le fil de guidage (14) transversalement dans et hors du canal à travers la fente (28).

9. Système de cathéter selon la revendication 8, caractérisé en ce que les moyens de guidage comprennent un organe de guidage (12) ayant des moyens séparés pour recevoir le fil de guidage (14) et le cathéter (10) et des moyens pour réunir le fil de guidage et le cathéter par guidage du fil de guidage transversalement à travers la fente (28) dans le cathéter et dans le canal du fil de guidage et pour séparer le fil de guidage et le cathéter par guidage du fil de guidage hors du canal transversalement à travers la fente (28).

10. Système de cathéter selon la revendication 9, caractérisé en ce que le fil de guidage (14) est disposé à l'intérieur du canal à distance des moyens de guidage (12) et s'étend transversalement hors du canal et du cathéter à proximité des moyens de guidage (12).

11. Système de cathéter selon la revendication 9, caractérisé en ce que les moyens de guidage sont réalisés et disposés de manière à effectuer ladite réunion et ladite séparation sous l'effet du déplacement relatif de l'organe de guidage (12) et d'au moins le fil de guidage (14) ou le cathéter (10).

12. Système de cathéter selon la revendication 11, caractérisé en ce que l'organe de guidage (12) comprend un corps traversé par un passage (48) pour le fil de guidage et par un passage (46) pour le cathéter, rencontrant le passage pour le fil de guidage, le passage pour le cathéter et le passage pour le fil de guidage se réunissant en un passage commun (44).

13. Système de cathéter selon la revendication 12, caractérisé par des moyens d'évasement (54) au voisinage de l'intersection desdits passages pour l'élargissement de la fente du corps de cathéter.

14. Système de cathéter selon la revendication 13, caractérisé en ce que les moyens d'évasement comprennent un organe écarteur (54) monté dans le corps de l'organe de guidage (12) à ladite intersection et sont prévus pour dépasser, à travers la fente (28) du cathéter dans l'intérieur du canal pour écarter les bords de la fente.

15. Système de cathéter selon la revendication 14, caractérisé en ce que l'écarteur (54) est situé à l'extrémité distale du passage de cathéter (46).

16. Système de cathéter selon la revendication 6, caractérisé en ce qu'un organe de guidage (12) est monté sur la cathéter et a des moyens pour recevoir un fil de guidage (14) et des moyens (12) pour réunir le fil de guidage et le cathéter en guidant le fil de guidage transversalement à travers la fente (28) du cathéter et vers l'intérieur du canal de fil de guidage et pour séparer le fil de guidage et le cathéter en guidant le fil de guidage transversalement hors du canal à travers ladite fente.

17. Système de cathéter selon la revendication 16, caractérisé en ce que le fil de guidage (14) est disposé à l'intérieur du canal à distance des moyens de guidage et s'étend transversalement hors du canal et du cathéter à proximité des moyens de guidage.

18. Système de cathéter selon la revendication 16, caractérisé en ce que les moyens de guidage sont réalisés et disposés de manière à effectuer cette réunion et cette séparation sous l'effet d'un déplacement relatif de l'organe de guidage vis à vis d'au moins le fil de guidage ou le cathéter.

19. Système de cathéter comprenant un corps de cathéter (10) souple et allongé, ayant une extrémité proximale et une extrémité distale et traversé longitudinalement par un canal (22), un accouplement de connexion (24) monté sur l'extrémité proximale du corps de cathéter, caractérisé en ce que le cathéter ne peut recevoir qu'un seul fil de guidage (14), en ce que le système permet un échange de cathéter sans échange des fils ou des fils de guidage, en ce que le corps du cathéter comporte au surplus un canal de fil de guidage (26) le traversant longitudinalement et une fente longitudinale linéaire unique (28) pratiquée dans le corps du cathéter (10) depuis un emplacement situé à proximité dudit accouplement (24) sur le corps de cathéter (10) et communiquant avec le canal de fil de guidage (26) pour permettre l'accès transversal au canal de fil de guidage (26) à travers le corps de cathéter (10), la fente s'étendant le long de la majeure portion de la longueur du corps de cathéter, et en ce que le système comprend au surplus un organe de guidage (12) monté sur le corps de cathéter (10) et ayant des moyens pour recevoir un fil de guidage (14) et des moyens pour réunir le fil de guidage et le cathéter par guidage du fil de guidage transversalement à travers la fente (28) dans le corps de cathéter et à l'intérieur du canal de fil de guidage et pour séparer le fil de guidage et le cathéter par guidage du fil de guidage transversalement hors du canal de fil de guidage (26) à travers ladite fente, ledit organe de guidage (12) étant réalisé et disposé de manière à effectuer cette réunion et cette séparation sous l'effet d'un déplacement relatif de l'organe de guidage par rapport à au moins le fil de guidage (14) ou le cathéter (10), ledit organe de guidage comprenant un corps traversé longitudinalement par un passage (48) pour le fil de guidage et un passage (46) pour le cathéter qui rencontre le passage pour fil de guidage (48), le passage pour cathéter et le passage pour fil de guidage se réunissant en un passage commun (44).

20. Système de cathéter selon la revendication 19, caractérisé par des moyens (54) voisins de l'intersection desdits passages pour évaser la fente (28) du corps de cathéter.

21. Système de cathéter selon la revendication 20, caractérisé en ce que les moyens d'évasement comprennent un organe écarteur (54) monté dans le corps (12) de l'organe de guidage à ladite intersection et se prolongeant à travers la fente (28) du cathéter jusque dans le canal pour écarter les bords de la fente.

22. Système de cathéter selon la revendication 21, caractérisé en ce que l'écarteur (54) est situé à l'extrémité distale du passage pour cathéter (46).

23. Système de cathéter selon la revendication 13 ou la revendication 22, caractérisé par un élément tubulaire (48) monté dans l'organe de guidage au voisinage de l'intersection des passages et ayant une extrémité distale (50) s'étendant vers le passage commun (44), l'élément tubulaire (48) ayant un plus petit diamètre que le passage commun (44) pour permettre au canal du fil de guidage du cathéter de s'envelopper autour de l'élément tubulaire, l'élément tubulaire étant prévu pour recevoir et guider le fil de guidage (14) dans l'intérieur du canal de fil de guidage du cathéter.
